# EUROPÄISCHE PATENTSCHRIFT

(11) **EP 1 205 209 B1**
(45) Veröffentlichungstag und Bekanntmachung des Hinweises auf die Patenterteilung: **01.09.2004**
(21) Anmeldenummer: 00124401.1
(22) Anmeldetag: 08.11.2000
(51) Int. Cl.: A61M 25/10

(54) **Kardioplegieballonkatheter**
Cardioplegia balloon catheter
Cathéter à ballonnet pour cardioplégie

(43) Veröffentlichungstag der Anmeldung: 15.05.2002
(73) Patentinhaber: Jostra AG, 72145 Hirrlingen (DE)
(72) Erfinder: Stumpp, Gerd, 72145 Hirrlingen (DE); Klietsch, Dietmar, 71083 Herrenberg (DE)
(74) Vertreter: Möbus, Daniela, Dr.-Ing.

(56) Entgegenhaltungen:
- US-A- 4 301 803
- US-A- 5 078 681
- US-A- 5 385 548
- US-A- 6 059 809

## Beschreibung

Die Erfindung betrifft eine Kanüle, insbesondere für den Einsatz als retrograde Kardioplegiekanüle, mit einem im Bereich ihrer Spitze angeordneten und mittels einer durch die Kanüle eingespeisten Flüssigkeit entfaltbaren Ballon zur Abdichtung des Raums zwischen der Kanüle und einem Gefäß und zur Positionierung der Kanüle im Gefäß. Der Ballon ist mit mehreren Öffnungen versehen, durch die die mittels der Kanüle eingespeise Flüssigkeit in das Gefäß austreten kann.

Solche Kanülen sind in der Herzchirurgie bereits bekannt. Sie werden als retrograde Kardioplegiekanülen eingesetzt, mit deren Hilfe Kardioplegieflüssigkeit zur Kühlung des Herzmuskels sowie zur Versorgung des Herzens mit Mineralien in den Körper des Patienten eingeführt wird. Die Kanülen weisen dazu an der Spitze eine Austrittsöffnung für die Kardioplegieflüssigkeit auf. Sie sind außerdem mit weiteren radialen Öffnungen im Bereich des Ballons versehen, durch die Kardioplegieflüssigkeit in den Ballon strömen kann, um diesen zu entfalten und die gewünschte Abdichtung des Gefäßes sowie die Positionierung der Kanüle zu erreichen. Die bekannten Kanülen haben jedoch den Nachteil, dass die vor dem Befüllen im Ballon stets vorhandene Luft nur ausgesprochen schlecht entweichen kann, sodass es bei der Entfaltung des Ballons zu Verzögerungen kommt.

Aus der US 6,059,809 ist eine Vorrichtung zum Einsetzen eines expandierbaren Stents in ein Gefäß bekannt. Diese Vorrichtung umfasst einen Ballon, dem eine Flüssigkeit zugeführt werden kann, um den Ballon auszudehnen, sodass er von innen an dem Gefäß anliegt. Der Ballon umfasst Öffnungen mit einer Art Ventilfunktion. Der Ballon wird mit der Flüssigkeit gefüllt und wenn er bei einem bestimmten Druck ganz entfaltet ist, öffnet sich die Ventilöffnung und die weiter zugeführte Flüssigkeit tritt zum proximalen Ende hin aus.

Der vorliegenden Erfindung liegt die Aufgabe zugrunde, eine Kanüle der eingangs beschriebenen Art dahin gehend zu verbessern, dass eine rasche Befüllung und Entfaltung des Ballons und eine rasche Verteilung von Kardioplegieflüssigkeit möglich ist.

Die Aufgabe wird durch eine Kanüle der eingangs genannten Art erfindungsgemäß dadurch gelöst, dass die Öffnungen über den Umfang des Ballons in dessen distalen Bereich angeordnet sind.

Die im Ballon vorhandenen Löcher ermöglichen das rasche und vollständige Entweichen der vorhandenen Luft, sobald die Flüssigkeit in den Ballon einströmt. Gleichzeitig dienen diese öffnungen auch als Austrittsöffnungen für die Kardioplegieflüssigkeit. Dadurch ist ein rasches Befüllen des Ballons und damit eine rasche Abdichtung des Gefäßes und sichere Positionierung der Kanüle im Gefäß gewährleistet.

Vorzugsweise können die öffnungen gleichmäßig über den Umfang des Ballons verteilt in dessen vorderem Bereich angeordnet sein. Damit wird eine äußerst gleichmäßige Verteilung der Kardioplegieflüssigkeit erreicht. Weitere Vorteile ergeben sich, wenn die öffnungen im Ballon die einzigen Austrittsöffnungen für die Flüssigkeit sind, d. h. keine weiteren Öffnungen im Bereich der Kanülenspitze für die Flüssigkeit vorhanden sind. Durch den Rückstau der Flüssigkeit von der Kanülenspitze her in den Ballon wird das Befüllen des Ballons weiter beschleunigt.

Für eine schonende Platzierung der Kanüle ist es außerdem zweckmäßig, wenn die Kanülenspitze abgerundet ist.

Nachfolgend wird ein bevorzugtes Ausführungsbeispiel einer erfindungsgemäßen Kanüle anhand der Zeichnung näher erläutert.

Es zeigen:
- Fig. 1: eine Seitenansicht einer erfindungsgemäßen Kanüle;
- Fig. 2: eine vergrößerte und geschnittene Darstellung der Kanüle aus Fig. 1 im Bereich II;
- Fig. 3: eine vergrößerte und geschnittene Darstellung der Kanüle aus Fig. 1 im Bereich III;
- Fig. 4: eine Vorderansicht auf den Ballon der Kanüle aus Fig. 1;
- Fig. 5: eine Seitenansicht des Ballons aus Fig. 4.

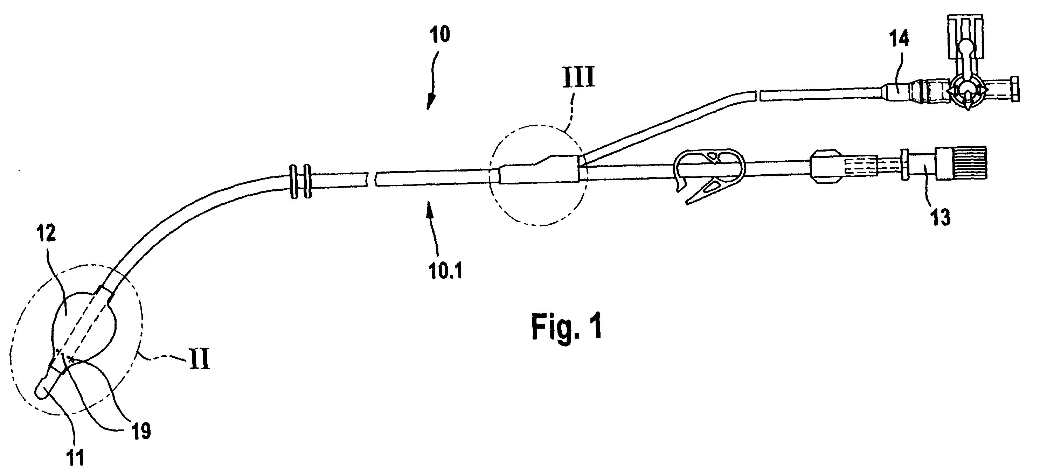

Die Kanüle 10 aus Fig. 1 weist im Bereich ihrer Spitze 11 einen Ballon 12 auf, mit dessen Hilfe die Kanüle 10 gegenüber einem Gefäß abgedichtet und positioniert werden kann. Am gegenüberliegenden Ende weist die Kanüle 10 einen Anschluss 13 zur Einspeisung einer Flüssigkeit, beispielsweise einer Kardioplegieflüssigkeit, auf. Außerdem ist ein weiterer Anschluss 14 zur Druckmessung vorgesehen, der im Bereich III in die eigentliche Kanüle 10 einmündet.

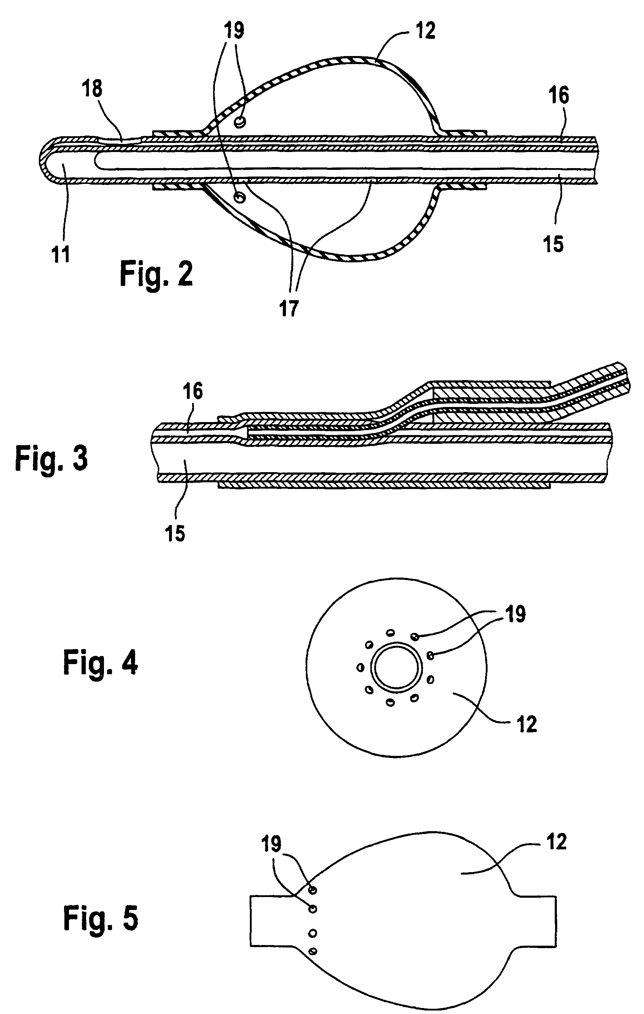

Wie die Fig. 2 und 3 zeigen, ist die Kanüle 10 in Längsrichtung in zwei getrennte Hohlräume 15 und 16 aufgeteilt, wobei der größere Hohlraum 15 der Führung der Flüssigkeit von der Anschlussstelle 13 her und der kleinere Hohlraum 16 zur Druckmessung vorgesehen ist. Wie Fig. 2 zeigt, weist das Kanülenrohr 10.1 im Bereich des Ballons 12 radiale öffnungen 17 auf, durch die die Flüssigkeit aus dem Hohlraum 15 in den Ballon 12 eintreten kann. Die Flüssigkeit zur Druckmessung tritt durch eine öffnung 18 im Bereich der Spitze 11 der Kanüle 10 ein.

Aus den Fig. 1, 4 und 5 ist zu erkennen, dass der Ballon 12 in seinem vorderen Bereich mit über seinen Umfang verteilten radialen öffnungen 19 versehen ist, durch die beim Befüllen des Ballons 12 zunächst die dort vorhandene Luft und anschließend die Flüssigkeit, insbesondere die Kardioplegieflüssigkeit, austreten kann. Da aus dem Hohlraum 15 im Kanülenrohr 10.1 außer den öffnungen 17 keine weiteren öffnungen nach außen führen, sind die Öffnungen 19 des Ballons 12 die einzigen öffnungen, durch die die Flüssigkeit in den Körper des Patienten ausströmen kann, wodurch gewährleistet ist, dass sich der Ballon 12 rasch befüllt, also eine schnelle Abdichtung zum Gefäß gegeben ist. Durch die gleichmäßige Verteilung der öffnungen 19 kommt es außerdem zu einer äußerst gleichmäbiegen Verteilung der Kardioplegieflüssigkeit, was bei einer einzelnen öffnung an der Spitze 11 der Kanüle 10 nicht gegeben wäre.

## Patentansprüche

1. Kanüle, insbesondere für den Einsatz als retrograde Kardioplegiekanüle, mit einem im Bereich ihrer Spitze (11) angeordneten und mittels einer durch die Kanüle (10) eingespeisten Flüssigkeit entfaltbaren Ballon (12) zur Abdichtung des Raums zwischen der Kanüle (10) und einem Gefäß und zur Positionierung der Kanüle (10) im Gefäß, wobei der Ballon (12) mit mehreren Öffnungen (19) versehen ist, durch die die mittels der Kanüle (10) eingespeiste Flüssigkeit in das Gefäß austreten kann, **dadurch gekennzeichnet, dass** die Öffnungen (19) über den Umfang des Ballons (12) verteilt in dessen distalen Bereich angeordnet sind.

2. Kanüle nach Anspruch 1, **dadurch gekennzeichnet, dass** die Öffnungen gleichmäßig verteilt sind.

3. Kanüle nach Anspruch 1 oder 2, **dadurch gekennzeichnet, dass** die öffnungen (19) im Ballon (12) die einzigen Austrittsöffnungen für die Flüssigkeit sind.

4. Kanüle nach einem der Ansprüche 1 bis 3, **dadurch gekennzeichnet, dass** die Kanülenspitze (11) abgerundet ist.

## Claims

1. Cannula, in particular for use as a retrograde cardioplegia cannula, having a balloon (12), disposed in the vicinity of its point (11), which can be expanded by means of a fluid fed through the cannula (10), said balloon (12) serving to seal off the space between the cannula (10) and a vessel and to position the cannula (10) in the vessel and being provided with a plurality of openings (19), through which the fluid fed in by means of the cannula (10) can emerge into the vessel, **characterized in that** the openings (19) are distributed over the circumference of the balloon (12) in its distal region.

2. Cannula according to Claim 1, **characterized in that** the openings are evenly distributed.

3. Cannula according to Claim 1 or 2, **characterized in that** the openings (19) in the balloon (12) are the only outlets for the fluid.

4. Cannula according to one of Claims 1 to 3, **characterized in that** the point (11) of the cannula is rounded off.

## Revendications

1. Canule, destinée en particulier à être utilisée comme canule rétrograde pour cardioplégie, comportant un ballon (12) disposé dans la zone de la pointe (11) de la canule et pouvant être gonflé au moyen d'un liquide introduit à travers la canule (10), pour rendre étanche l'espace entre la canule (10) et un vaisseau et pour positionner la canule (10) dans le vaisseau, le ballon (12) étant pourvu de plusieurs orifices (19) à travers lesquels le liquide introduit au moyen de la canule (10) peut s'échapper dans le vaisseau, **caractérisée en ce que** les orifices (19) sont répartis sur le pourtour du ballon (12), dans sa zone distale.

2. Canule selon la revendication 1, **caractérisée en ce que** les orifices sont régulièrement répartis.

3. Canule selon la revendication 1 ou 2, **caractérisée en ce que** les orifices (19) ménagés dans le ballon (12) sont les seuls orifices de sortie du liquide.

4. Canule selon l'une des revendications 1 à 3, **caractérisée en ce que** la pointe (11) de la canule est arrondie.
